# EUROPEAN PATENT SPECIFICATION

(11) **EP 1 390 114 B1**
(45) Date of publication and mention of the grant of the patent: **29.12.2010**
(21) Application number: 01930754.5
(22) Date of filing: 27.04.2001
(51) Int. Cl.: B01D 17/038

(54) **APPARATUS WITH VORAXIAL SEPARATOR AND ANALYZER**
VORRICHTUNG MIT VAS (VORAXIAL SEPARATOR) UND ANALYSATOR
APPAREIL DOTE D'UN ANALYSEUR ET D'UN SEPARATEUR VORAXIAL

(43) Date of publication of application: 25.02.2004
(73) Proprietor: Enviro Voraxial Technology, Inc., Fort Lauderdale, FL 33309 (US)
(72) Inventor: DIBELLA, Alberto, Fort Lauderdale ,FL 33309 (US); ANTHONY, Michael, M., Coral Springs, FL 33065 (US)
(74) Representative: Trinks, Ole
(86) International application number: PCT/US2001/013348
(87) International publication number: WO 2003/070349

(56) References cited:
- US-A- 3 517 821
- US-A- 4 478 712
- US-A- 5 084 189
- US-A- 5 840 007
- US-A- 5 904 840

## Description

### BACKGROUND OF THE INVENTION

### 1. Field of the Invention:

The present invention relates generally to the field of oil reclamation devices. More specifically the present invention relates to a voraxial separator and analyzer apparatus for separating and reclaiming oil from water, such as in the aftermath of a tanker ship oil spill, and for separating and reclaiming pure water from industrial contaminants. The apparatus includes a composite fluid passing tube containing a first set of rotation vanes referred to as rotation acceleration vanes connected to vane rotation means. The set of rotation acceleration vanes is oriented to deliver angular acceleration to a composite fluid within the tube about the longitudinal axis of the tube to a given rotational speed at which the component fluids making up the composite fluid separate into radially stratified layers according to their densities. A second set of rotation vanes referred to as rotation maintenance vanes is inventively provided within the tube downstream of the rotation acceleration vanes which preferably provide just enough angular force to sustain the rotation of the stratified component fluids against the force of friction at the tube wall and against fluid viscosity, and thus to prevent turbulence resulting from transition to a lower angular speed which would result in loss of the fluid stratification.

A second key feature of the present invention is the provision of an analyzer system including an integrated package uniting two motors with two systems. A feedback loop in communication with a computer monitor and a method of spinning fluid are provided. A tachometer is connected to the set.of rotation maintenance vanes to measure fluid rotation rate, and is wired to the feedback loop.

### 2. Description of the Prior Art:

Millions of gallons of precious fuel oil are obtained from wells each year, and yet great quantities of sludge and water often mix with this fuel oil and must be removed to make the oil usable. In some areas of the world drinking water is in short supply and must be separated from contaminants. Major sources of drinking water contamination are chemicals and particulate matter mingling with cleaning water used during metal finishing and other chemical etching processes. Separation of such contaminants represents an economic burden and more often a loss of precious drinking water. In Europe the rising cost of water and water processing plants which use existing purification methods such as the ion exchange systems has become a major concern for government and industry alike. Industrial wastes and fuels entering water supplies have also had profound environmental and economic impacts on coastal fishing grounds. In order to rapidly purify, capture and store pure water, and to recover lost fuel and other recyclable contaminants, the water and contaminant mixtures must be pumped from storage reservoirs and separated. A voraxial separation system capable of achieving such separation is disclosed generally in U.S. Patent Number 5,084,189 issued to the present applicant. Many of the specifications of the present separator are described in U.S. Patent Number 5,084,189. A limitation of this prior separator, however, has been the prospect of turbulence within the flow stream following angular acceleration and radial component stratification, resulting from transition to lower rotational speed, causing some intermixing of stratified layers. US-A-4 478 712 discloses a centrifugal separator for immiscible fluid of different specific gravities comprising a tubular body and at least two separate means for driving the fluid in rotation. Extraction ducts are provided to withdraw the separated fluid and means to feed back remaining fractions of the mixture.

It is thus an object of the present invention to provide a composite fluid separation apparatus which separates a composite fluid medium consisting of one or more component fluids including contaminants of different densities by centrifugal acceleration and stratification of the medium, which is induced by forces imparted by a set of rotation acceleration vanes to cause the medium to spin axially within a composite fluid passing tube, creating a force vortex action on the medium body which, in turn causes the spun medium to separate into its component fluids and contaminants at different radii corresponding to their respective densities, the separated medium being transported axially along the cylindrical pipe and separated by radially positioned pipe intakes. The lighter fluid is forced by free vortex action and by Bernoulli pressure forces into a tight cylindrical flow along the central axis of the spinning medium, while the heavier particles are spun along the outer radii of the spinning medium.

It is another object of the present invention to provide such an apparatus which, in addition to voraxially accelerating and stratifying component fluids for independent removal from the flow stream, maintains the necessary stratification speed against deceleration turbulence until component fluid removal is accomplished.

It is another object of the present invention to provide such an apparatus which automatically monitors and controls the rotation acceleration and maintenance vane speeds necessary to cause and maintain fluid separation through use of sensors and a feedback loop tied to a computer.

It is another object of the present invention to provide such an apparatus which includes sensory and detection means to monitor the concentration of contaminants in both the contaminant take off pipe and the fluid take off pipe.

It is another object of the present invention to provide such an apparatus which provides a method of monitoring the amount of unseparated fluid within the different radially separated layers, and to compute feedback signals to correctly determine and control the rotation speed of the separator and achieve maximum separation results.

It is another object of the present invention to provide such a method of monitoring the amount of unseparated fluids and to compute a feedback signal in order to control a bleed valve and thus to modify the density of the medium by injection of air into the medium before the separation. The air stream serves to modify the density of the centrally separated fluid stream and thus to minimize the loss of the higher density fluids to the central stream.

It is another object of the present invention to provide such an apparatus which efficiently separates the various components of contaminated fluid, such as water mixed with mud, oil and slimes, and to differentiate these components into their various densities, so that they can be removed from the flow stream, for the purposes of recovery and removal of the components.

It is still another object of the present invention to provide such an apparatus which relies on a computerized analyzer system that efficiently separates the various components of contaminated fluid for the purposes of recovery and removal of the components.

It is finally an object of the present invention to provide such an apparatus which relies on a feedback loop with a computerized analyzer system that efficiently determines the concentrations of the various components of contaminated fluid for the purposes of recovery and/or removal of the components.

The foregoing and other objects of the invention are achieved by provision of an axial flow type pump for separating immiscible fluids having different specific gravities and a discharge manifold fluid connected to the fluid pump for drawing of the fluid having the heavier specific gravity; a sensor attached to the inlet flow into the separator; a sensor attached to the outlet flow of the separator; a sensor attached to the central light fluid take off manifold; a signal generating means from the sensors; means for transporting the signal generated in the sensors to a signal processing analyzer, and computing a feedback control signal; means for using the computed feedback signal to accurately control the speed of the separator and the amount of air intake into the separator.

The present invention is an apparatus for the efficient and consistent separation of the components of a fluid with a built in integrated analyzer for determining the concentration of each component of the separated fluid. The purpose of the invention is to separate unwanted components of the fluid by means of a voraxial separator and to analyze the separated components thereof with a complete integrated control system. The present invention has the advantages of an integrated and complete system that embodies all of the elements of the analyzer and the voraxial separator in one unit. The apparatus consists of a voraxial separator of unique design, which relies on the free rotation of a fluid rather than the forced rotation of a fluid, and thus is not subject to the various hydrodynamic limitations of current centrifugal separators on the market, the apparatus also consists of a sensor probe, where the sensor probe relies on the varying scatter fields of electromagnetic radiation by density variations of the probe; a means of receiving the electromagnetic radiation through fiber-optic cables; and a means of computing the density field of the fluid at a given cross section; and a motor controller means for adequately controlling the separation rate of the voraxial separator. One distinguishing design of the present apparatus from the prior art is the use of specially designed guiding surfaces and specially shaped vanes that propel and control the centrifugal flow of the fluid during the separation stage.

### SUMMARY OF THE INVENTION

The present invention accomplishes the above-stated objectives, as well as others, as may be determined by a fair reading and interpretation of the entire specification.

A voraxial separator and analyzer apparatus is provided, including a composite fluid passing tube having a tube upstream segment and a tube downstream segment; a rotation acceleration vane structure rotationally mounted within the tube upstream segment and being oriented to deliver angular acceleration to a composite fluid within the tube about the longitudinal axis of the tube for separating the fluid into radially stratified component layers within the tube in the order of component density and to propel the fluid toward and through the tube downstream segment; an acceleration vane drive mechanism drivably connected to the rotation acceleration vane structure; a rotation maintenance vane structure rotationally mounted within the tube downstream segment and being oriented to deliver angular acceleration to a composite fluid within the tube about the longitudinal axis of the tube for maintaining the rotational velocity of the fluid imparted by the rotation acceleration vane structure against friction losses; and a maintenance vane drive mechanism drivably connected to the rotation maintenance vane structure.

The apparatus preferably additionally includes a second rotation maintenance vane structure within the tube downstream segment downstream of the rotation maintenance vane structure. The rotation acceleration vane structure optionally includes a belt driven impeller. The rotation maintenance vane structure optionally includes a belt driven impeller.

The apparatus preferably additionally includes a fluid probe extending into the tube downstream of the rotation acceleration structure positioned to detect fluid composition at several radial locations within the tube and to generate probe signals, and a fluid analyzer computer receiving and analyzing the probe signals and displaying fluid composition information.

The apparatus preferably still additionally includes a first speed control mechanism controlling the rotational rate of the acceleration vane drive mechanism; a second speed control mechanism controlling the rotational rate of the maintenance vane drive mechanism; a feedback loop mechanism interconnecting the fluid analyzer computer mechanism and the first speed control mechanism and the second speed control mechanism; so that the fluid analyzer computer causes the first speed control mechanism to alter the rate of rotation of the rotation acceleration vane structure to achieve and maintain radial layer component separation and to alter the rate of rotation maintenance vane structure rotation to achieve and maintain the rotation speed of the fluid emerging from the rotation acceleration vane structure with automatic control and feedback.

### BRIEF DESCRIPTION OF THE DRAWINGS

Various other objects, advantages, and features of the invention will become apparent to those skilled in the art from the following discussion taken in conjunction with the following drawings, in which:
FIGURE 1 is a perspective view of the complete apparatus, showing primarily the exterior of the housing and the inlet port and controls.
FIGURE 2 is a perspective view of the housing with the forward housing wall removed to reveal the voraxial tube, rotational acceleration and maintenance vane structures.
FIGURE 3 is a perspective view of the apparatus as in FIGURE 1, but shown from the opposing, discharge side.
FIGURE 4 is a perspective view of the voraxial separation assembly, including the voraxial tube having the intake and output tube end flanges and showing the rotational acceleration and maintenance vane structures and drive means, and also showing a layer removal tube and a probe.
FIGURE 5 is another perspective view of the voraxial separation assembly, showing two of the probes, one downstream of the rotation acceleration vane structure and the other downstream of the rotation maintenance vane structure.
FIGURE 6 is a perspective view of the apparatus.
FIGURE 7 is another perspective view of the apparatus with a top portion of the housing removed to reveal some of the voraxial separation assembly.
FIGURE 8 is a sectional view of part of the voraxial tube and of the probe light reflector mirrors and lenses, and of a layer removal tube.
FIGURE 9 is a different perspective view of the voraxial separation assembly, showing the rotational acceleration and maintenance vane structures and the two probes and fiber-optic filaments and two layer removal tubes.
FIGURE 10 is a perspective broken away view of the one of the rotational vane structures.
FIGURE 11 is another perspective view of the structure of FIGURE 10.
FIGURE 12 is a perspective view of the apparatus with the housing front wall removed.
FIGURE 13 is an operational flow chart.
FIGURE 14 is an operational flow chart.

### DETAILED DESCRIPTION OF THE PREFERRED EMBODIMENTS

As required, detailed embodiments of the present invention are disclosed herein; however, it is to be understood that the disclosed embodiments are merely exemplary of the invention which may be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure.

Reference is now made to the drawings, wherein like characteristics and features of the present invention shown in the various FIGURES are designated by the same reference numerals.

### First Preferred Embodiment

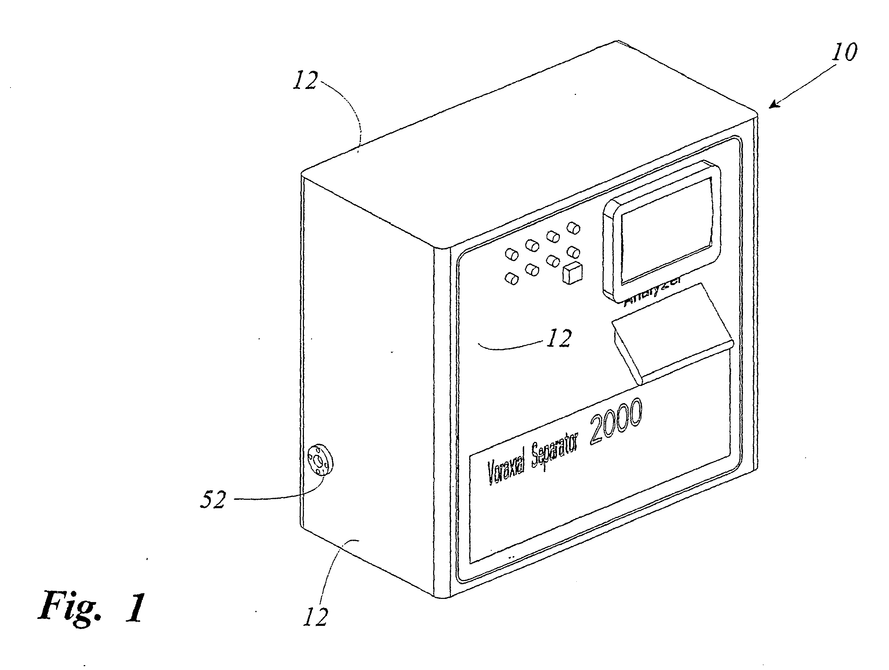

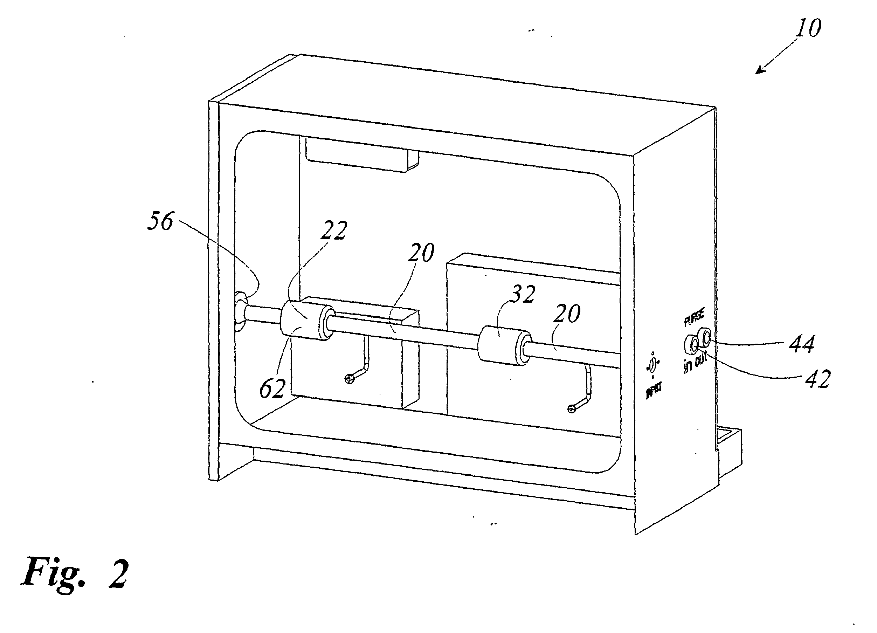

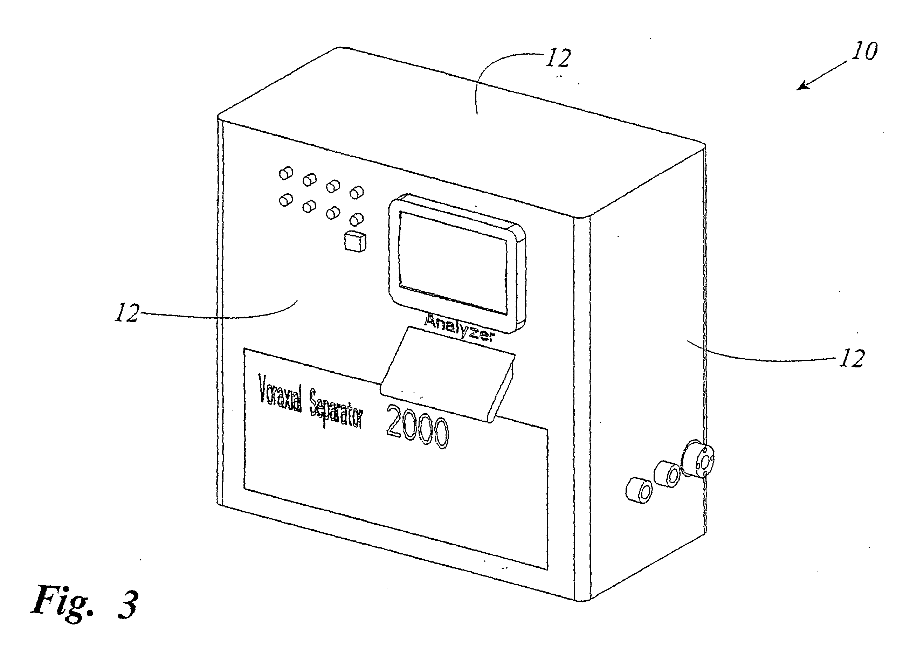

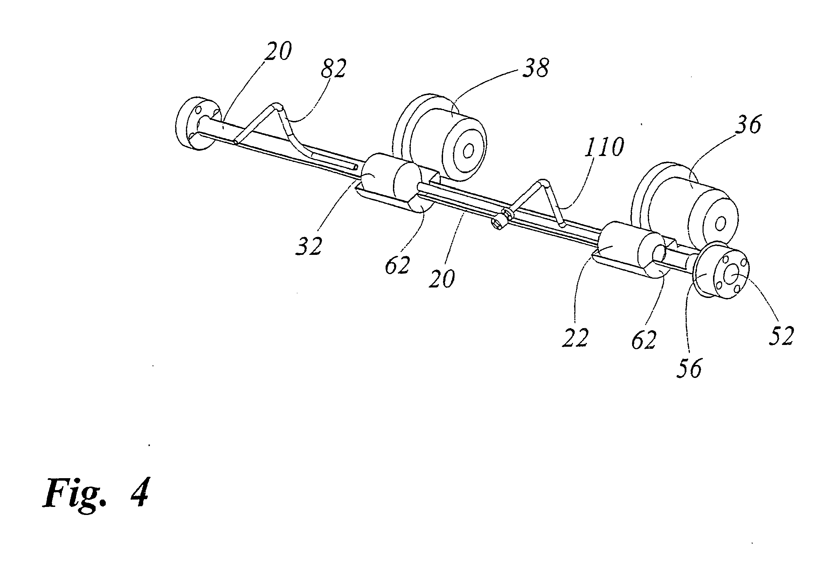

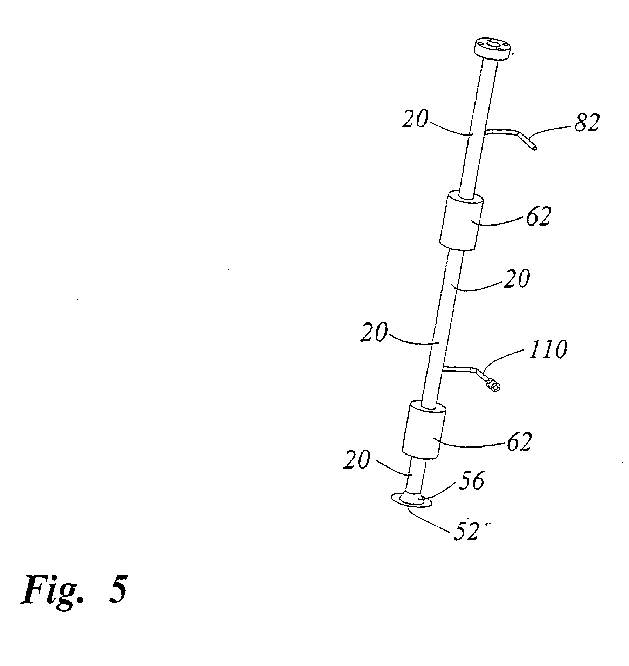

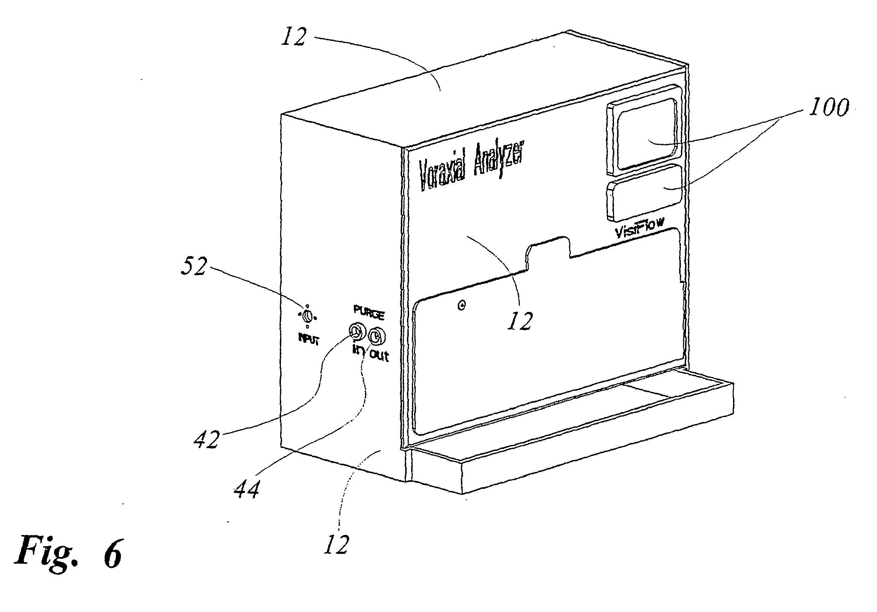

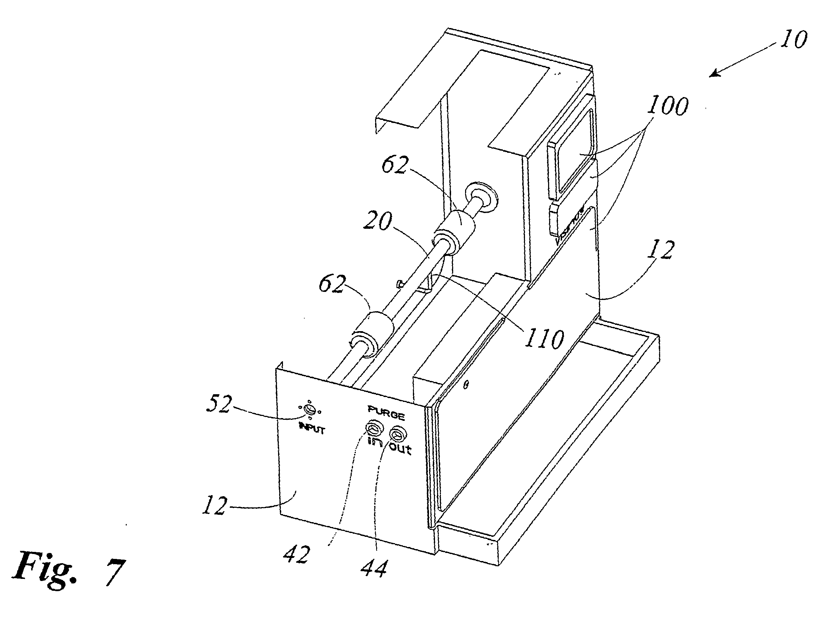

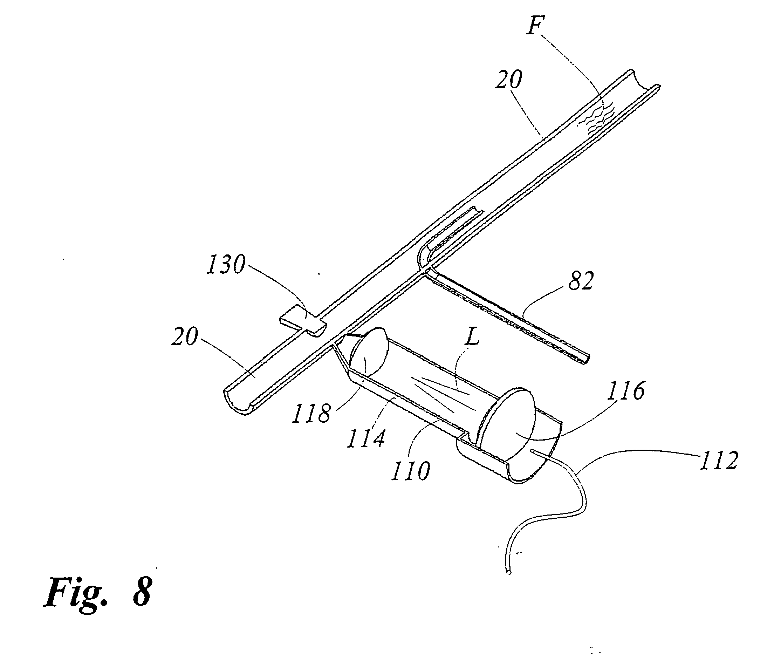

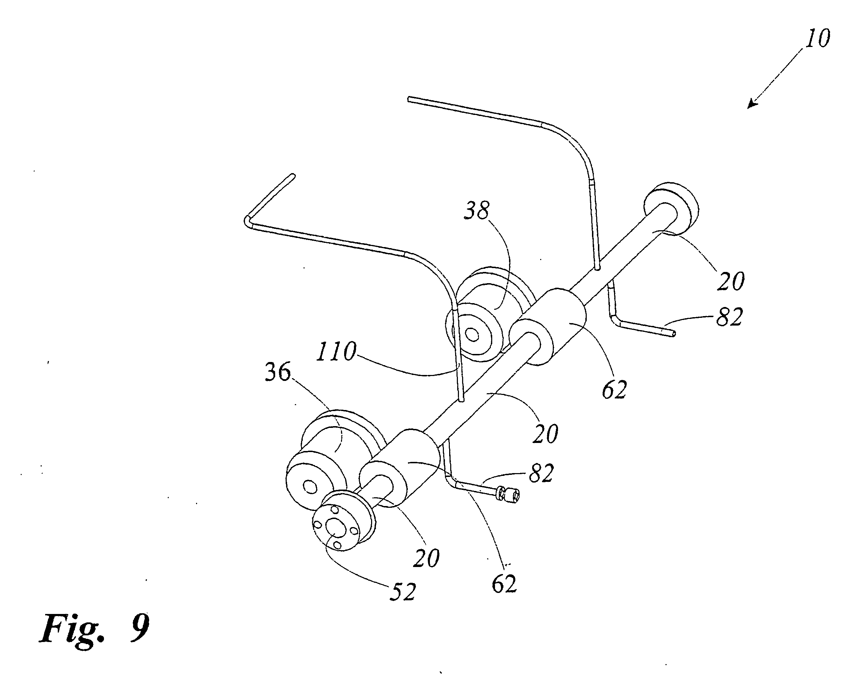

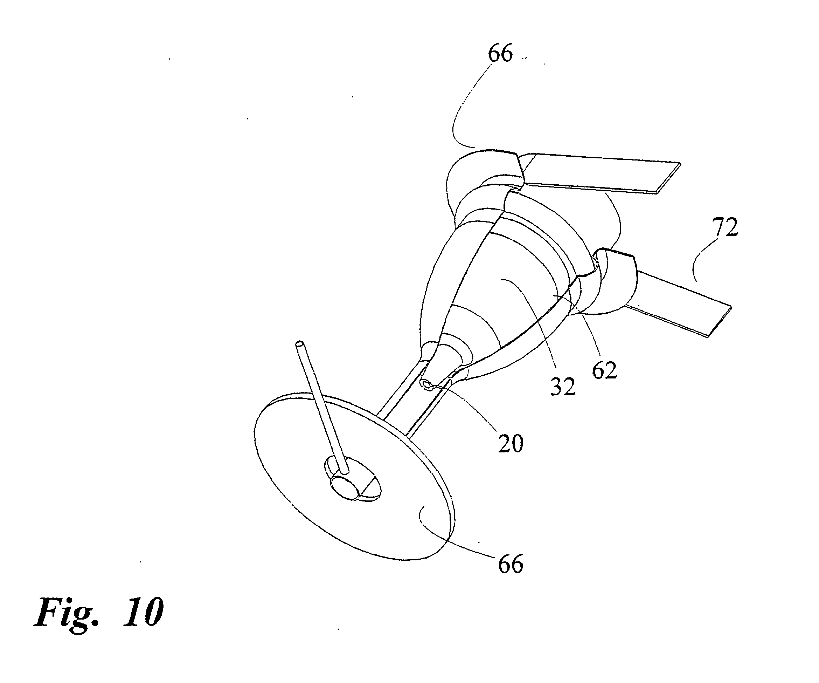

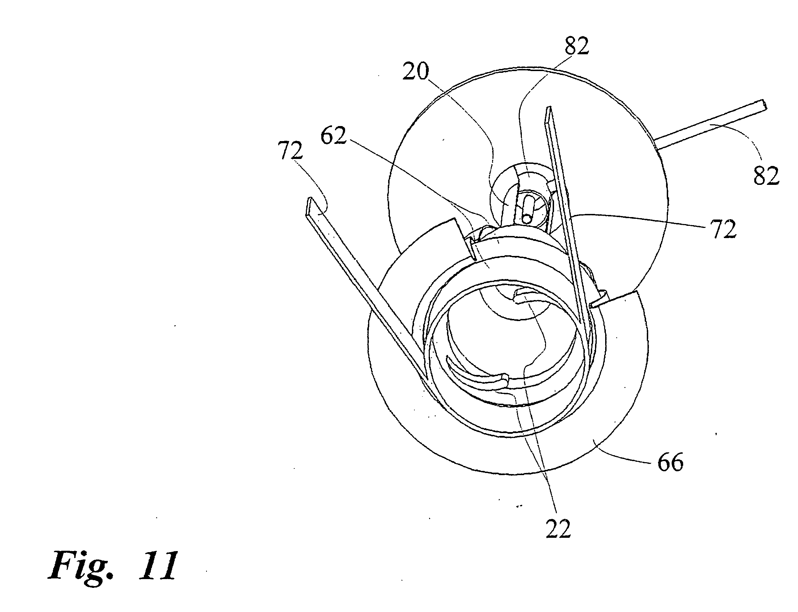

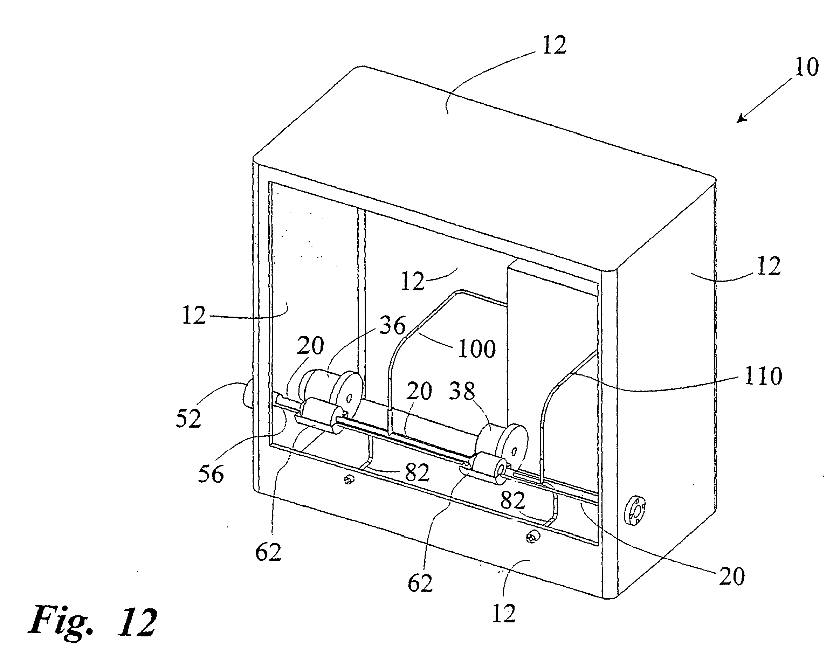

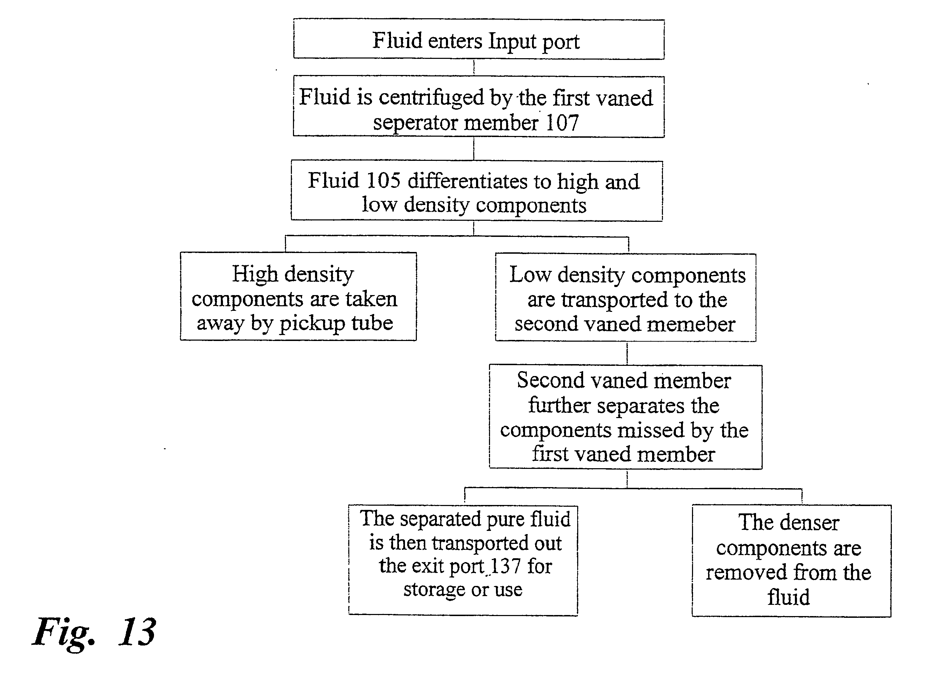

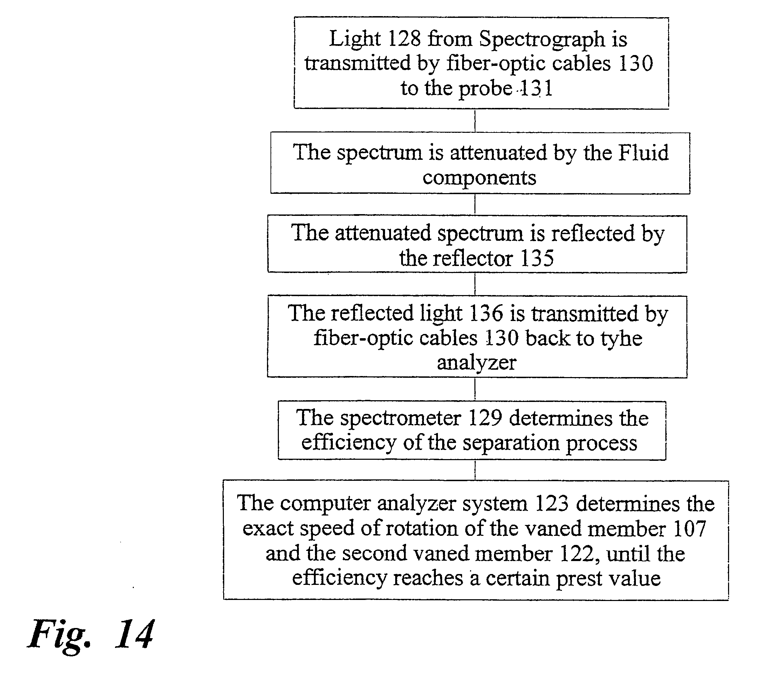

Referring to FIGURES 1-14, a voraxial separator and analyzer apparatus 10 is disclosed. Apparatus 10 includes a composite fluid passing voraxial tube 20 containing a first set of rotation vanes referred to as rotation acceleration vane member 22 connected to vane rotation means 24. The set of rotation acceleration vane member 22 is oriented to deliver angular acceleration to a composite fluid within the tube 20 about the longitudinal axis of the tube 20 to a given rotational speed at which the component fluids making up the composite fluid separate into radially stratified layers. The set of rotation acceleration vane member 22 also deliver linear, axial velocity to the composite fluid to propel the composite fluid through the tube 20 while it is being separated. A second set of rotation vanes referred to as rotation maintenance vane member 32 is inventively provided within the tube 20 downstream of the set of acceleration vane member 22. Rotation maintenance vane member 32 preferably provide just enough angular force to sustain the rotation of the stratified component fluids against the force of friction at the tube wall and against internal friction of the fluid resulting from the fluid viscosity, and thus prevent turbulence resulting from transition to a lower rotational speed which would result in loss of some of the fluid stratification. Absent rotation maintenance vane member 32, the fluid stream would reach a point of transition flow a certain distance beyond the acceleration vanes at which reverse flow in the form of angular deceleration and crossflow between stratified layers would result, causing as well as resulting from associated turbulence. A turbulence-free transition from forced vortex to free vortex is thus provided by rotation maintenance vane member 32. Additional sets of rotation maintenance vanes (not shown) are optionally provided periodically further downstream, depending upon the length of tube 20, as is necessary to maintain stratification speed and prevent deceleration turbulence. The set of rotation acceleration vanes and set or sets of rotation maintenance vanes are each preferably belt driven impellers as described below, but may take other forms.

A second key feature of the present invention is the provision of an analyzer and feedback system including an integrated package uniting two motors with two systems. A feedback loop in communication with a computer monitor and a method of spinning fluid are provided. A tachometer is connected to the set of rotation maintenance vanes to measure fluid rotation rate, and is wired to the feedback loop.

Apparatus 10 includes a housing 12, which is designed to meet the requirements of fire resistance in petrochemical and chemical processing plants. The housing 12 retains all the components of apparatus 10 within a sealed environment. A purge gas input port 42 is provided to permit the interior of the apparatus 10 components including tube 20 to be cleaned and purged from all explosive gases before electric power is connected to the apparatus 10 components. In a petrochemical plant, this purging permits apparatus 10 to be used without fear of causing an explosion when electrical power is supplied to apparatus 10. The purge gas is regulated at a fixed input pressure greater than atmospheric pressure, so that during the initial operation of apparatus 10, inert gas such as nitrogen is introduced throughout the inside of apparatus 10, and permitted to purge and displace any contaminating gas that may have accumulated inside apparatus 10. A second output purge port 44 is provided to remove the contaminant gases from inside apparatus 10. Thus a stable gas flow is established between purge ports 42 and 44 which serves to clean apparatus 10 during operation.

Apparatus 10 has a fluid input port 52 for receiving any flowable fluid F for voraxial separation. Port 52 can be a bolt-on-flange of standard design in the industry. Port 52 is the connection member to the entry port for voraxial tube 20. Voraxial tube 20 is designed to permit very smooth flow of the fluid F, so that as little turbulence as possible is created within the flowing fluid. A guiding cone 56 permits the fluid to accelerate into the fluid drive housing 12.

During operation fluid input port 52 is connected to the intake pipe (not shown) of the fluid reservoir which is to be processed and separated. A portion of the voraxial tube 20 is sealingly connected to a lager fluid drive housing 62. Inside fluid drive housing 62, is an acceleration vane member 22 which is designed to be powered by a fluid drive acceleration vane motor 36. The acceleration vane member 22 rotates relative to the fluid drive housing 62 through a set of centering sealing bearings 64. Motor 36 is connected to a rotational drive flange 66 of vane member 22 by either gears (not shown) or by a fine friction belt 72. Motor 36 drives belt 72 which then imparts the rotational energy to acceleration vane member 22. Vane member 22 couple with fluid inside voraxial tube 20. The rotation rate of acceleration vane member 22 is measured by a tachometer attached to drive flange 66. A torque sensor is also provided on drive flange 66 to determine the torque needed to spin the fluid and its components by the acceleration vane member 22 at a given rotation rate.

The acceleration vane member 22 couples with fluid F, creating a low pressure vortex that rapidly pulls fluid through the vane member 22. The rapid rotation of the fluid F results in unhindered radial density differential separation of the fluid and its components by reason of the centrifugal forces acting on the fluid components A-C. Fluid components A, B and C represent the extreme density ranges of the components of the fluid F. Since the rotation of the fluid is a forced rotation, a rapid density gradient is experienced by the fluid F, causing the heavier components of fluid F to move toward the periphery of the voraxial tube 20, while the lighter components are pushed toward the center of the voraxial field. The forced vortex configuration only occurs when the fluid is being propelled and spun in the acceleration vane member 22. The situation becomes drastically different when the free vortex is permitted to form and be maintained downstream of the acceleration vane member 22. Just a few centimeters downstream of the forced vortex tail, a free vortex starts to form and the fluid starts to become dynamically stable as a free vortex. Although the pure fluid has a constant density, a pressure field develops that creates a central field of low pressure in the free vortex. The free vortex then starts to pull the heavier particles and fluid components into the center of the voraxial tube 20. A first collector tube 82 removes most of the heavier components of the fluid, filtering about 99.9 percent of the solids and heavy fluid components in the fluid F. After this filtering, fluid F is essentially free from most of the solid and chemical components of heavier density than fluid F. The clean fluid stream then enters a maintenance vane member 32. Maintenance vane member 32 is essentially of similar design to acceleration vane member 22, and is powered by motor 38. However the rotation rate of maintenance vane member 32 is very well controlled to match the free rotation rate of the fluid F.

In conventional centrifuges, the crossover from a forced vortex field to free vortex field causes some turbulence that can de-stabilize the transition region of the denser components. The present apparatus 10 is thus advantageously designed to solve this problem by first applying the centrifuge technique on the fluid F with acceleration vane member 22, and then rapidly pulsing maintenance vane member 32 to impart rotational vectors on the fluid F, so that during each pulse, the friction loss of rotational energy of fluid F is compensated for by the energy imparted on fluid F by maintenance vane member 32. Instead of turbulence building up downstream of acceleration vane member 22, maintenance vane member 32 causes the fluid to maintain a free vortex configuration without ever entering a transition zone in maintenance vane member 32. Thus a finer separation is always maintained by the apparatus 10. Apparatus 10 is equipped with a computer and analyzer system 100, which receives information from the acceleration vane member 22 and maintenance vane member 32, and then calculates and controls the rotation rate of motor 36, and motor 38 from data received from the tachometers, and torque sensors. Thus by relaxing the rotation rate of maintenance vane member 32 to exactly match the rotation of the fluid that has been imparted in a forced vortex by the acceleration vane member 22, the vane member 32 does not impart any energy to the fluid other than that needed to overcome loses due to friction. The computer analyzer system 100 thus serves to just drive the fluid F at the desired separation speed while minimizing the forced vortex conditions on the fluid F. Thus the crossover or transition from the forced vortex to free vortex is minimized considerably. This in turn maximizes the separation efficiencies of the apparatus 10. In addition, the acceleration vane member 22 may also be controlled to impart only rotation energy to make up for friction losses. Thus, after the initial rapid rotation of the fluid F, the fluid is then rotating in essentially a free vortex mode.

In order to determine the accuracy of the separation, a fi.ber-optic probe 110 its attached to the side wall of voraxial tube 20 to determine the concentration of the fluid components that are being separated. Near-infrared (NIR) light L is generated by a spectrophotometer or a spectrometer 112 contained within the apparatus 10. The light L is transmitted through bi-directional fiber-optic cables 112 to fiber-optic probe 110. Fiber-optic probe 110 comprises a tubular housing 114 which holds focusing lenses 116 and 118. The light L passes through the fiber-optic probe 110, and is collimated and projected by lenses 116 and 118, through fluid F and onto a mirrored stainless steel reflector 130. Deflector 130 is conveniently positioned diametrically opposite a fiber-optic probe 110 on voraxial tube 20. When the light L passes through the fluid F, a unique spectrum is absorbed by the fluid F at that location. This spectrum is then used by computer analyzer system 100 to determine the concentrations of the fluid components present at that location. The reflected light L is again captured by the bi-directional fiber-optic cable 112 and transported to the spectrograph 112. The spectral characteristics at the scanning point of the fluid F can thus be determined by means of computer analyzer 100. The preferred computer analyzer 100 is described in U.S. Patent Number 5,489,900 to Anthony. Fiber optic, probe 110 is also described in U.S. Patent Number 5,044,755 to Thus the efficiency of the separation process can be accurately determined by singular integrated apparatus 10, described in this invention.

While the invention has been described, disclosed, illustrated and shown in various terms or certain embodiments or modifications which it has assumed in practice, the scope of the invention is not intended to be, nor should it be deemed to be, limited thereby.

## Claims

1. A voraxial separator and analyzer apparatus, comprising:
a composite fluid passing tube (20) having a tube upstream segment and a tube downstream segment;
rotation acceleration vane means (22) rotationally mounted within said tube upstream segment and being oriented to deliver angular acceleration to a composite fluid (F) within said tube (20) about the longitudinal axis of said tube for separating the fluid into radially stratified component layers within said tube in the order of component density and to propel the fluid toward and through said tube downstream segment;
acceleration vane drive means (36) drivably connected to said rotation acceleration vane means (22);
first rotation maintenance vane means (32) rotationally mounted within said tube downstream segment and being oriented to deliver angular acceleration to a composite fluid (F) within said tube (20) about the longitudinal axis of said tube for maintaining the rotational velocity of the fluid imparted by said rotation acceleration vane means against friction losses;
and maintenance vane drive means (38) drivably connected to said first rotation maintenance vane means (32),
fluid probe means (110) extending into said tube (20) downstream of said rotation acceleration means (22) positioned to detect fluid composition at a plurality of radial locations within said tube and to generate probe signals, and fluid analyzer computer means (100) receiving and analyzing said probe signals and displaying fluid composition information, and
first speed control means controlling the rotational rate of said acceleration vane drive means (36); second speed control means controlling the rotational rate of said maintenance vane drive means (38); feedback loop means interconnecting said fluid analyzer computer means (100) and said first speed control means and said second speed control means;
such that said fluid analyzer computer means (100) causes said first speed control means to alter the rate of rotation of said rotation acceleration vane means (22) to achieve and maintain radial layer component separation with automatic control and feedback,
**characterized in that**
the analyser computer means (100) are adapted to control the rotation rate of the acceleration vane derive means (36) and the maintenance vane drive means (38) from data received from tachometers and torque sensors of the acceleration vane means (22) and maintenance vane means (32) so that the rotational acceleration vane means (22) create a forced vortex in said tube upstream segment and thereby also creating a free vortex within said tube downstream segment, and said rotation maintenance vane means (32) sustaining said free vortex against degeneration and thus sustaining the stratification of the component layers.

2. The apparatus of claim 1, additionally comprising a second rotation maintenance vane means (32) within said tube downstream segment downstream of said first rotation maintenance vane means (32).

3. The apparatus of claim 1, wherein said rotation acceleration vane means (229 comprises a belt driven impeller.

4. The apparatus of claim 1, wherein said rotation maintenance vane means (32) comprises a belt driven impeller.

5. The apparatus of claim 1, wherein said fluid probe means comprises a fiber-optic probe (110) and a spectrophotometer (112) connected to said fiber-optic probe with a bi-directional fiber-optic cable (112) and a reflector element (130) within said tube (20) opposite said fiber-optic probe (110); such that said spectrophotometer generates light (L), and the light passes through said fluid to said reflector element (130), whereupon a unique spectrum of the light is absorbed by the fluid (F), and whereupon the ramining light is reflected by said reflector element to said fiber-optic probe for analysis by said fluid analyzer computer means (100).

## Patentansprüche

1. VAS (Voraxial Separator)- und Analysatorvorrichtung, aufweisend:
ein zusammengesetztes Fluiddurchflußrohr (20) mit einem stromaufwärtigen Rohrsegment und einem stromabwärtigen Rohrsegment,
Rotationsbeschleunigungs-Schaufelmittel (22), welche drehbar in dem stromaufwärtigen Rohrsegment montiert und ausgerichtet sind, um einem zusammengesetzten Fluid (F) in dem Rohr (20) eine Winkelbeschleunigung um die Längsachse des Rohres zum Trennen des Fluides in radial geschichtete Komponentenschichten in dem Rohr in der Reihenfolge der Komponentendichte zu verleihen, und um das Fluid zu dem und durch das stromabwärtige Rohrsegment zu treiben;
Schaufel-Beschleunigungsmittel (36), die antreibbar mit den Rotationsbeschleunigungs-Schaufelmitteln (22) verbunden sind, erste Rotationsaufrechterhaltungs-Schaufelmittel (32), die drehbar innerhalb des stromabwärtigen Rohrsegments angeordnet sind und ausgerichtet sind, einem zusammengesetzten Fluid (F) in dem Rohr (20) eine Winkelbeschleunigung um die Längsachse des Rohrs zum Aufrechterhalten der Rotationsgeschwindigkeit des Fluides, welche durch die Rotationsbeschleunigungs-Schaufelmittel verliehen wurde, gegen Reibungsverluste, zu verleihen;
und Aufrechterhaltungs-Schaufelantriebsmittel (38), welche antreibbar mit den ersten Rotationsaufrechterhaltungs-Schaufelmittein (32) verbunden sind, Fluidsondenmittel (110), welche sich in das Rohr (20) stromabwärts der Rotationsbeschleunigungsmittel (22) erstrecken, die positioniert sind, um die Fluidzusammensetzung an einer Vielzahl von radialen Stellen innerhalb des Rohres zu erfassen und Sondensignale zu erzeugen,
und Fluidanalysator-Computermittel (100), welche die Sondensignale erhalten und analysieren und eine Fluidzusammensetzungsinformation anzeigen, und erste Geschwindigkeitssteuermittel, welche die Rotationsrate der Beschleunigungs-Schaufelantriebsmittel (36) steuern; zweite Geschwindigkeitssteuermittel, welche die Rotationsrate der Aufrecherhaltungs-Schaufelantriebsmittel (38) steuern;
Rückkopplungsschleifenmittel, welche die Fluidanalysator-Computermittel (100) und die ersten Geschwindigkeitssteuermittel und die zweiten Geschwindigkeits-Steuermittel verbinden;
so dass die Fluidanalysator-Computermittel (100) bewirken, dass die ersten Geschwindigkeitssteuermittel die Rotationsrate der Rotationsbeschleunigungs-Schaufelmittel (22) ändern, um eine Komponententrennung in radiale Schichten mit automatischer Steuerung und Feedback zu erzielen und aufrechtzuerhalten,
**dadurch gekennzeichnet,**
**dass** die Analysator-Computermittel (100) ausgelegt sind, die Rotationsrate der Beschleunigungs-Schaufelantriebsmittel (36) und der Aufrechterhaltungs-Schaufelantriebsmittel (38) aus Daten zu steuern, welche von Tachometern und Drehmomentsensoren der Beschleunigungs-Schaufelmittel (22) und Aufrechterhaltungs-Schaufelmittel (32) erhalten werden, so dass die Rotationsbeschleunigungs-Schaufelmittel (22) einen erzwungenen Wirbel in dem stromaufwärtigen Rohrsegment erzeugen und **dadurch** auch einen freien Wirbel in dem stromabwärtigen Rohrsegment, und dass die Rotationsaufrechterhaltungs-Schaufelmittel (32) den freien Wirbel gegen Degeneration aufrechterhalten und so die Schichtenbildung der Komponentenschichten aufrechterhalten.

2. Vorrichtung nach Anspruch 1, zusätzlich aufweisend ein zweites Rotationsaufrechterhaltungs-Schaufelmittel (32) in dem stromabwärtigen Rohrsegment stromabwärts des ersten Aufrechterhaltungs-Schaufelmittels (32).

3. Vorrichtung nach Anspruch 1, wobei das Rotationsbeschleunigungs-Schaufelmittel (22) ein riemengetriebenes Antriebsrad aufweist.

4. Vorrichtung nach Anspruch 1, wobei das Rotationsaufrechterhaltungs-Schaufelmittel (32) ein riemengetriebenes Antriebsrad aufweist.

5. Vorrichtung nach Anspruch 1, wobei das Fluidsondenmittel eine faseroptische Sonde (110) und ein Spektralphotometer (112), das mit der faseroptischen Sonde mit einem bidirektionalen faseroptischen Kabel (112) verbunden ist, und ein Reflektorelement (130) in dem Rohr (20) der faseroptischen Sonde (110) gegenüberliegend aufweist; so dass das Spektralphotometer Licht (L) erzeugt und das Licht durch das Fluid zum Reflektorelement (130) verläuft, worauf ein einzigartiges Spektrum des Lichts durch das Fluid (F) absorbiert wird und worauf das verbleibende Licht durch das Reflektorelement zur faseroptischen Sonde zur Analyse durch das Fluidanalysator-Computermittel (100) reflektiert wird.

## Revendications

1. Appareil analyseur et séparateur voraxial, comprenant :
un tube prévu pour le passage de liquide composite (20) ayant un segment amont de tube et un segment aval de tube ;
des moyens d'ailette d'accélération de rotation (22) fixés de façon tournante à l'intérieur dudit segment amont de tube et étant orientés pour apporter une accélération angulaire à un liquide composite (F) à l'intérieur dudit tube (20) autour de l'axe longitudinal dudit tube pour séparer le liquide en couches d'éléments stratifiés dans le plan radial à l'intérieur dudit tube dans l'ordre de densité des éléments pour propulser le liquide en direction dudit segment aval de tube et à travers lui ;
des moyens d'entraînement d'ailette d'accélération (36) reliés en fonctionnement auxdits moyens d'ailette d'accélération de rotation (22) ;
des premiers moyens d'ailette de maintien de rotation (32) fixés de façon tournante à l'intérieur dudit segment aval de tube et étant orientés pour apporter l'accélération angulaire à un liquide composite (F) à l'intérieur dudit tube (20) autour de l'axe longitudinal dudit tube pour maintenir la vitesse de rotation du liquide donnée par lesdits moyens d'ailette d'accélération de rotation afin de compenser les pertes de frottement ; et
des moyens d'entraînement d'ailette d'entretien (38) reliés en fonctionnement auxdits premiers moyens d'ailette de maintien de rotation (32) ;
des moyens de sondage de liquide (110) s'étendant dans ledit tube (20) en aval desdits moyens d'accélération de rotation (22) positionnés pour détecter la composition du liquide à une pluralité d'emplacements radiaux à l'intérieur dudit tube et pour produire des signaux de sondage, et des moyens informatiques d'analyse de liquide (100) recevant et analysant lesdits signaux de sondage et affichant les informations de composition du liquide ; et
des premiers moyens de commande de la vitesse de rotation desdits moyens d'entraînement d'ailette d'accélération (36) ; des seconds moyens de commande de la vitesse de rotation desdits moyens d'entraînement d'ailette d'entretien (38) ; des moyens de boucle de rétroaction interconnectant lesdits moyens informatiques d'analyse de liquide (100) et lesdits premiers moyens de commande de vitesse et lesdits seconds moyens de commande de vitesse ;
de telle sorte que lesdits moyens informatiques d'analyse de liquide (100) amènent lesdits premiers moyens de commande de vitesse à modifier la vitesse de rotation desdits moyens d'ailette d'accélération de rotation (22) pour obtenir et maintenir la séparation des éléments de couche radiale en utilisant la commande automatique et la rétroaction ;
**caractérisé en ce que** :
les moyens informatiques d'analyse (100) sont adaptés pour commander la vitesse de rotation des moyens d'entraînement d'ailette d'accélération (36) et des moyens d'entraînement d'ailette d'entretien (38) à partir des données reçues de tachymètres et pour coupler les capteurs des moyens d'ailette d'accélération (22) et les moyens d'ailette d'entretien (32) de façon à ce que les moyens d'ailette d'accélération de rotation (22) créent un vortex forcé dans ledit segment amont de tube, créant ainsi également un vortex libre à l'intérieur dudit segment aval de tube, et lesdits moyens d'ailette de maintien de rotation (32) empêchant ledit vortex libre de se dégrader, soutenant ainsi la stratification des couches d'éléments.

2. Appareil selon la revendication 1, comprenant en outre des seconds moyens d'ailette de maintien de rotation (32) à l'intérieur dudit segment aval de tube en aval desdits premiers moyens d'ailette de maintien de rotation (32).

3. Appareil selon la revendication 1, dans lequel lesdits moyens d'ailette d'accélération de rotation (229) comprennent une turbine entraînée par courroie.

4. Appareil selon la revendication 1, dans lequel lesdits moyens d'ailette de maintien de rotation (32) comprennent une turbine entraînée par courroie.

5. Appareil selon la revendication 1, dans lequel lesdits moyens de sondage de liquide comprennent une sonde à fibre optique (110) et un spectrophotomètre (112) relié à ladite sonde à fibre optique avec un câble à fibre optique bidirectionnel (112) et un élément réfléchissant (130) à l'intérieur dudit tube (20) opposé à ladite sonde à fibre optique (110) ; de telle sorte que ledit spectrophotomètre émet de la lumière (L) et que la lumière traverse ledit liquide en direction dudit élément réfléchissant (130), faisant qu'un spectre du lumière unique est absorbé par le liquide (F) et que la lumière restante est réfléchie par ledit élément réfléchissant en direction de ladite sonde à fibre optique pour être analysée par lesdits moyens informatiques d'analyse de liquide (100).
